# EUROPEAN PATENT APPLICATION

(11) **EP 2 578 562 A1**
(43) Date of publication of application: **10.04.2013**
(21) Application number: 11786306.8
(22) Date of filing: 20.05.2011
(51) Int. Cl.: C07C 37/72, C07C 39/15

(54) **CYCLIC COMPOUND PURIFICATION METHOD**

(30) Priority: 26.05.2010 JP 2010120444
(71) Applicant: Mitsubishi Gas Chemical Company, Inc., Tokyo 100-8324 (JP)
(72) Inventor: OKADA, Yu, Hiratsuka-shi Kanagawa 254-0016 (JP); HAYASHI, Hiromi, Hiratsuka-shi Kanagawa 254-0016 (JP); ECHIGO, Masatoshi, Hiratsuka-shi Kanagawa 254-0016 (JP)
(74) Representative: HOFFMANN EITLE
(86) International application number: PCT/JP2011/002825
(87) International publication number: WO 2011/148603

(57) **Abstract**

An industrially advantageous purification method for a cyclic compound with a particular structure is provided.

A purification method for a cyclic compound, including a step of contacting a solution containing a cyclic compound with a particular structure and an organic solvent with water or an acidic aqueous solution.

## Description

### TECHNICAL FIELD

The present invention relates to a purification method for a cyclic compound, particularly to an industrially advantageous purification method for a cyclic compound with a reduced metal content.

### RELATED ART

As a candidate of a low molecular weight resist material, a positive type resist composition using a low molecular weight cyclic polyphenol compound with a structure of having an acid dissociable functional group introduced into at least one phenolic hydroxyl group as a main component (see Patent Documents 1 to 9, Non-Patent Documents 1 and 2), or an alkali developing type resist composition using a low molecular weight cyclic polyphenol compound as a main component (see Patent Document 9 and Non-Patent Document 3) have been disclosed.

These low molecular weight cyclic polyphenol compounds are expected to provide a resist pattern with small molecular size, high resolution and small roughness due to low molecular weight. Also, a low molecular weight cyclic polyphenol compound provides high heat resistance even with low molecular weight, by having a rigid cyclic structure in the backbone thereof.

Moreover, for a resist composition, in addition to basic characteristics such as sensitivity, resolution and roughness, particularly metal content is an important characteristic point for improving the yield. More specifically, since a metal remains in a pattern drawn to decrease the electric property of a semiconductor in case of a resist composition with a large metal content is used, the metal content is required to be reduced.

As a production method for a cyclic compound with a reduced metal content, a method of contacting a mixture containing a cyclic compound and an organic solvent with an ion exchange resin, a method of filtering it with a filter, and the like are possible. However, in the method using an ion exchange resin, when various metal ions are contained, selection of an ion exchange resin is difficult and there are a problem in that removal is difficult depending on the kind of metals, a problem in that removal of a nonionic metal is difficult, and furthermore, a problem in that the running cost is large. On the other hand, in the method of filtering it with a filter, there is a problem in that removal of ionic metal is difficult. Therefore, an industrially advantageous purification method for a cyclic compound with a reduced metal content has been desired to be established.

### RELATED DOCUMENTS

### Patent Documents

- Patent Document 1:: JP-A-H11-153863
- Patent Document 2:: JP-A-H11-322656
- Patent Document 3:: JP-A-2002-328473
- Patent Document 4:: JP-A-2003-321433
- Patent Document 5:: JP-A-2005-170902
- Patent Document 6:: JP-A-2006-276459
- Patent Document 7:: JP-A-2006-276742
- Patent Document 8:: JP-A-2007-8875
- Patent Document 9:: JP-A-2009-173623

### Non-Patent Documents

Non-Patent Document 1: Seung Wook Chang et al., "Materials for Future Lithography", Proc. SPIE, Vol. 5753, p.1
Non-Patent Document 2: Daniel Bratton et al., "Molecular Glass Resists for Next Generation Lithography", Proc. SPIE, Vol. 6153, 61531D-1
Non-Patent Document 3: T. Nakayama, M. Nomura, K. Haga, M. Ueda: Bull. Chem. Soc. Jpn., 71, 2979 (1998)

### SUMMARY OF THE INVENTION

### Problem to be Solved by the Invention

The object of the invention is to provide an industrially advantageous purification method for a cyclic compound with a reduced metal content.

### Means for Solving the Problem

The inventors have, as a result of devoted examinations to solve the above problem, discovered that by contacting a solution containing a cyclic compound and an organic solvent with water or an acidic aqueous solution, the contents of various metals significantly decrease, and have reached the present invention.
More specifically, the present invention is as follows.
1. A purification method for a cyclic compound with a reduced metal content, including a step of contacting a solution containing a cyclic compound represented by the following formula (1) and an organic solvent with water or an acidic aqueous solution. 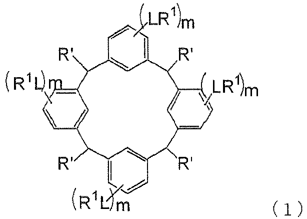 (In the formula (1), L is independently a divalent group selected from the group consisting of a single bond, a linear or branched alkylene group of 1 to 20 carbons, a cycloalkylene group of 3 to 20 carbons, an arylene group of 6 to 24 carbons, -O-, OC(=O)-, -OC(=O)O-, -N(R⁵)-C(=O)-, -N(R⁵)-C(=O)O-, -S-, -SO-, -SO₂- and arbitrary combinations thereof, R¹ is independently an acid dissociable functional group selected from the group consisting of an alkyl group of 1 to 20 carbons, a cycloalkyl group of 3 to 20 carbons, an aryl group of 6 to 20 carbons, an alkoxyl group of 1 to 20 carbons, a cyano group, a nitro group, a hydroxyl group, a heterocyclic group, a halogen atom, a carboxyl group, an acyl group of 2 to 20 carbons, an alkylsilyl group of 1 to 20 carbons, a substituted methyl group of 2 to 20 carbons, a 1-substituted ethyl group of 3 to 20 carbons, a 1-substituted-n-propyl group of 4 to 20 carbons, a 1-branched alkyl group of 3 to 20 carbons, a silyl group 1 to 20 carbons, an acyl group of 2 to 20 carbons, a 1-substituted-alkoxyalkyl group of 2 to 20 carbons, a cyclic ether group of 2 to 20 carbons, an alkoxycarbonyl group of 2 to 20 carbons and an alkoxycarbonylalkyl group, or a hydrogen atom, R' is independently an alkyl group of 2 to 20 carbons or a group represented by the following formula 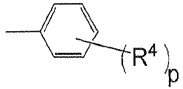 wherein, R⁴ is independently a functional group selected from the group consisting of an alkyl group of 1 to 20 carbons, a cycloalkyl group of 3 to 20 carbons, an aryl group of 6 to 20 carbons, an alkoxy group of 1 to 20 carbons, a cyano group, a nitro group, a hydroxyl group, a heterocyclic group, a halogen atom, a carboxyl group and an alkylsilyl group of 1 to 20 carbons, or an acid dissociable functional group selected from the group consisting of a substituted methyl group of 2 to 20 carbons, a 1-substituted ethyl group of 3 to 20 carbons, a 1-substituted-n-propyl group of 4 to 20 carbons, a 1-branched alkyl group of 3 to 20 carbons, a silyl group 1 to 20 carbons, an acyl group of 2 to 20 carbons, a 1-substituted-alkoxyalkyl group of 2 to 20 carbons, a cyclic ether group of 2 to 20 carbons, an alkoxycarbonyl group of 2 to 20 carbons and an alkoxycarbonylalkyl group, R⁵ is a hydrogen atom or an alkyl group of 1 to 10 carbons, m is an integer of 1 to 4, and p is an integer of 0 to 5.)
2. A purification method according to the above item 1, further including a step of conducting an extraction treatment of the cyclic compound.
3. A purification method according to the above item 2, further including a step of separating the solution phase containing the cyclic compound and the organic solvent and the water phase after the extraction treatment.
4. A purification method according to the above item 3, further including a step of recovering a solution containing the cyclic compound and the organic solvent.
5. A purification method according to any of the above items 1 to 4, wherein the acidic aqueous solution is an aqueous solution of one or more kinds of mineral acids selected from the group consisting of hydrochloric acid, sulfuric acid, nitric acid and phosphoric acid.
6. A purification method according to any of the above items 1 to 4, wherein the acidic aqueous solution is an aqueous solution of one or more kinds of organic acids selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid and trifluoroacetic acid.
7. A purification method according to any of the above items 1 to 4, wherein the acidic aqueous solution is an aqueous solution of at least one kind of polycarboxylic acid selected from oxalic acid, tartaric acid and citric acid.
8. A purification method according to the above item 6, wherein the acidic aqueous solution is an aqueous solution of oxalic acid.
9. A purification method according to any of the above items 1 to 8, wherein the organic solvent is toluene, 2-heptanone, cyclohexanone, cyclopentanone, methyl isobutyl ketone, or ethyl acetate.
10. A purification method according to the above item 9, wherein the organic solvent is cyclohexanone.
11. A purification method according to any of the above items 1 to 10, wherein the cyclic compound represented by the formula (1) is selected from the group consisting of each compound represented by the following formula (2). 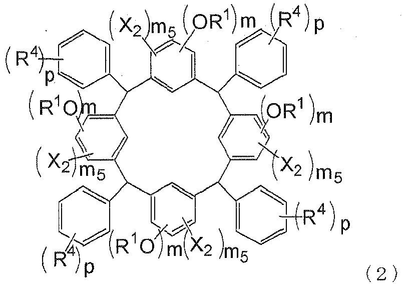 (In the formula (2), R¹, R⁴, p and m are the same as above. X₂ is a hydrogen atom or a halogen atom, m₅ is an integer of 0 to 3, and m+m₅=4.)
12. A purification method according to the above item 11, wherein the compound represented by the formula (2) is selected from the group consisting of each compound represented by the following formula (3). 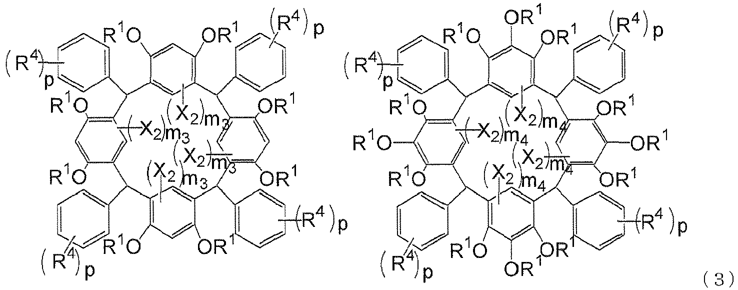 (In the formula (3), R¹, R⁴, X₂ and p are the same as above. m₃ is 2, and m₄ is 1.)
13. A purification method according to the above item 11, wherein the compound represented by the formula (2) is selected from the group consisting of each compound represented by the following formula (4). 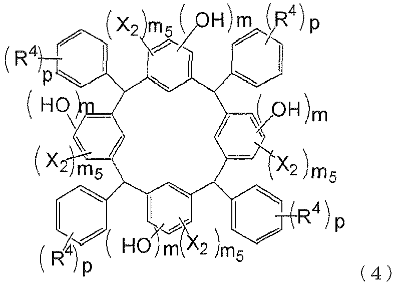 (In the formula (4), R⁴, X₂, m, m₅ and p are the same as above.)
14. A purification method according to the above item 13, wherein the compound represented by the formula (4) is selected from the group consisting of each compound represented by the following formula (5). 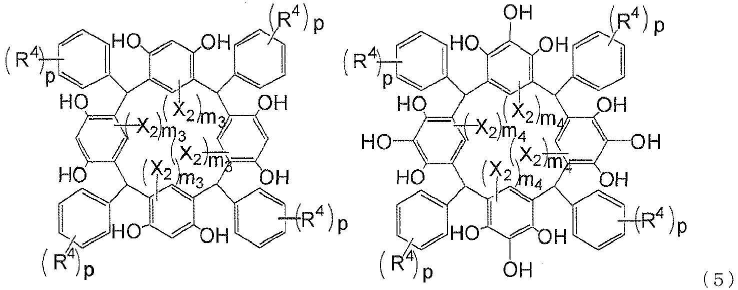 (In the formula (5), X₂, R⁴, m₃, m₄ and p are the same as above.)
15. A purification method according to the above item 14, wherein the compound represented by the formula (5) is selected from the group consisting of each compound represented by the following formulae (6-1) and (6-2). 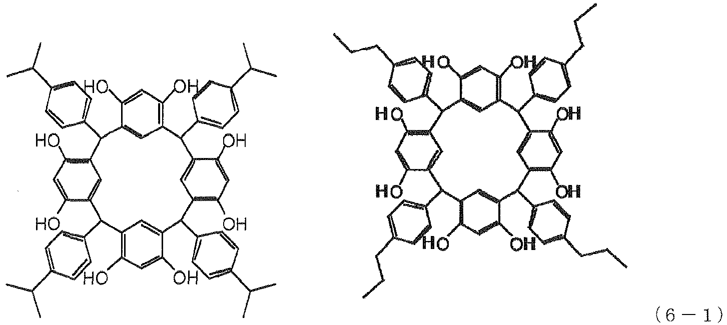 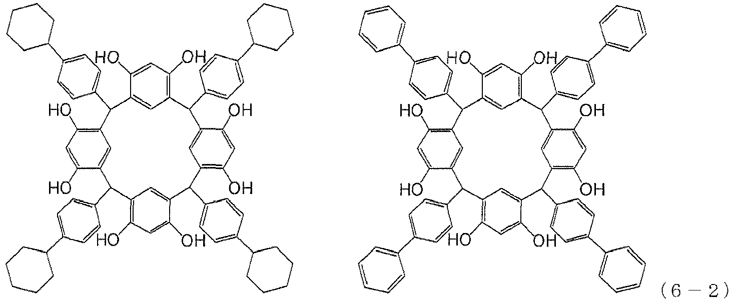
16. A purification method according to any of the above items 1 to 10, wherein the cyclic compound represented by the formula (1) is selected from the group consisting of each compound represented by the following formula (7). 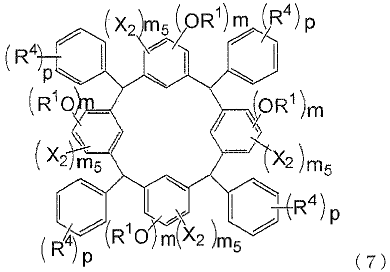 (In the formula (7), R¹, R⁴, p and m are the same as above. However, at least one R¹ thereof is an acid dissociable functional group. X₂ is a hydrogen atom or a halogen atom, m₅ is an integer of 0 to 3, and m+m₅=4.)
17. A purification method according to the above item 16, wherein the compound represented by the formula (7) is selected from the group consisting of each compound represented by the following formula (8). 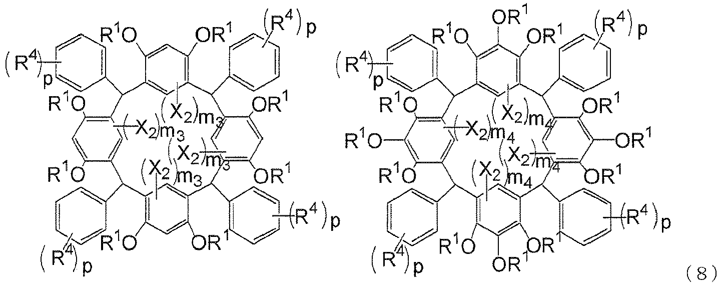 (In the formula (8), R¹, R⁴, X₂ and p are the same as above. However, at least one R¹ thereof is an acid dissociable functional group. m₃ is 2, and m₄ is 1.)
18. A purification method according to the above item 17, wherein the compound represented by the formula (8) is selected from the group consisting of each compound represented by the following formulae (9-1) and (9-2). 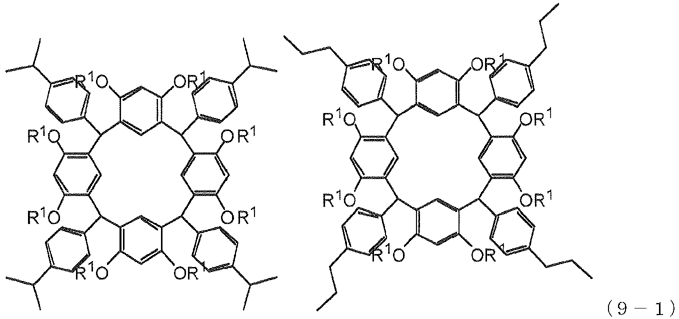 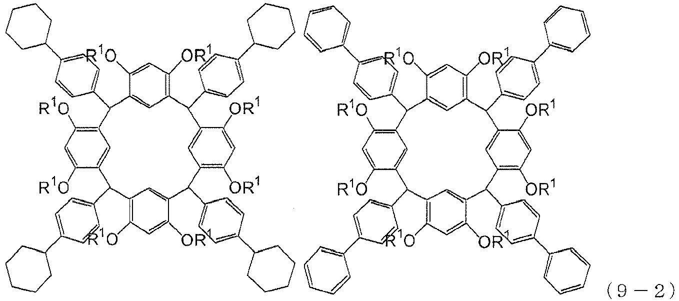
(In the formulae (9-1) and (9-2), R¹ is the same as above. However, at least one R¹ thereof is an acid dissociable functional group.)

### Effect of the Invention

According to the present invention, it is possible to provide a purification method for a cyclic compound having the contents of various metals significantly reduced.

### MODE FOR CARRYING OUT THE INVENTION

The present invention will be described in details below.
The present invention is a purification method for a cyclic compound with a reduced metal content by dissolving a cyclic compound in an organic solvent, contacting the solution with water or an acidic aqueous solution, and thereby transferring the metal content contained in the solution to the water phase.

The cyclic compound used in the present invention is a cyclic compound represented by the following formula (1).

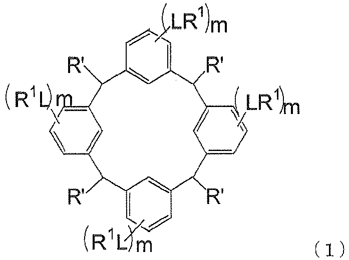

(In the formula (1), L is independently a divalent group selected from the group consisting of a single bond, a linear or branched alkylene group of 1 to 20 carbons, a cycloalkylene group of 3 to 20 carbons, an arylene group of 6 to 24 carbons, -O-, - OC(=O)-, -OC(=O)O-, -N(R⁵)-C(=O)-, -N(R⁵)-C(=O)O-, -S-, -SO-, -SO₂- and arbitrary combinations thereof, R¹ is independently a functional group selected from the group consisting of an alkyl group of 1 to 20 carbons, a cycloalkyl group of 3 to 20 carbons, an aryl group of 6 to 20 carbons, an alkoxyl group of 1 to 20 carbons, a cyano group, a nitro group, a hydroxyl group, a heterocyclic group, a halogen atom, a carboxyl group, an acyl group of 2 to 20 carbons, an alkylsilyl group of 1 to 20 carbons and derivatives thereof, an acid dissociable functional group selected from the group consisting of a substituted methyl group of 2 to 20 carbons, a 1-substituted ethyl group of 3 to 20 carbons, a 1-substituted-n-propyl group of 4 to 20 carbons, a 1-branched alkyl group of 3 to 20 carbons, a silyl group 1 to 20 carbons, an acyl group of 2 to 20 carbons, a 1-substituted-alkoxyalkyl group of 2 to 20 carbons, a cyclic ether group of 2 to 20 carbons, an alkoxycarbonyl group of 2 to 20 carbons and an alkoxycarbonylalkyl group, or a hydrogen atom, R' is independently an alkyl group of 2 to 20 carbons or a group represented by the following formula 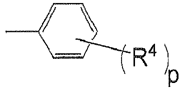 wherein, R⁴ is independently a functional group selected from the group consisting of an alkyl group of 1 to 20 carbons, a cycloalkyl group of 3 to 20 carbons, an aryl group of 6 to 20 carbons, an alkoxy group of 1 to 20 carbons, a cyano group, a nitro group, a hydroxyl group, a heterocyclic group, a halogen atom, a carboxyl group and an alkylsilyl group of 1 to 20 carbons, or an acid dissociable functional group selected from the group consisting of a substituted methyl group of 2 to 20 carbons, a 1-substituted ethyl group of 3 to 20 carbons, a 1-substituted-n-propyl group of 4 to 20 carbons, a 1-branched alkyl group of 3 to 20 carbons, a silyl group 1 to 20 carbons, an acyl group of 2 to 20 carbons, a 1-substituted-alkoxyalkyl group of 2 to 20 carbons, a cyclic ether group of 2 to 20 carbons, an alkoxycarbonyl group of 2 to 20 carbons and an alkoxycarbonylalkyl group, R⁵ is a hydrogen atom or an alkyl group of 1 to 10 carbons, m is an integer of 1 to 4, and p is an integer of 0 to 5.)

The cyclic compounds represented by the formula (1) preferably include the following compounds.

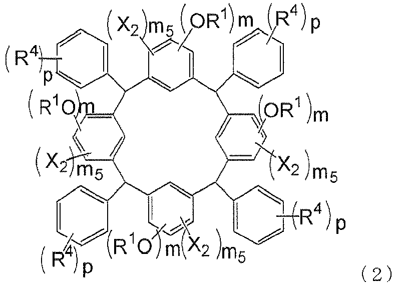

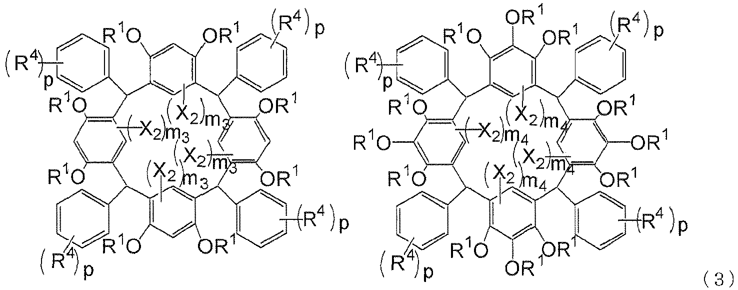

(In the formulae (2) and (3), R¹, R⁴, p and m are the same as above. X₂ is a hydrogen atom or a halogen atom, m₅ is an integer of 0 to 3, and m+m₅=4, m₃ is 2, and m₄ is 1.)

When a cyclic compound does not have any of the above acid dissociable functional groups therein, it is cyclic compound (A) which is useful as a main component of a negative type resist composition, and when it has one or more of the above acid dissociable functional groups therein, it is cyclic compound (B) which is useful as a main component of a positive type resist composition.

In the present invention, as the cyclic compound (A), the following are preferable. Cyclic compounds (A) represented by the following formulae (4) and (5) are preferable.

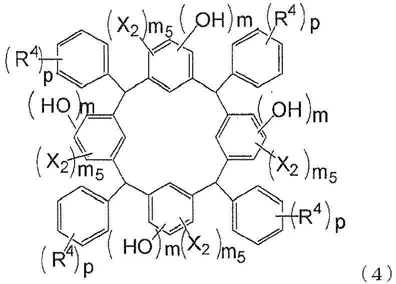

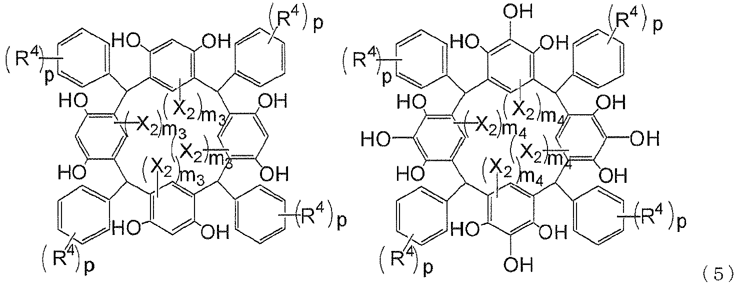

(In the formulae (4) and (5), R⁴, X₂, m, m₃, m₄, m₅ and p are the same as above.)

In the present invention, as the above cyclic compound (A), compounds represented by the following formula (6-1) or (6-2) are more preferable.

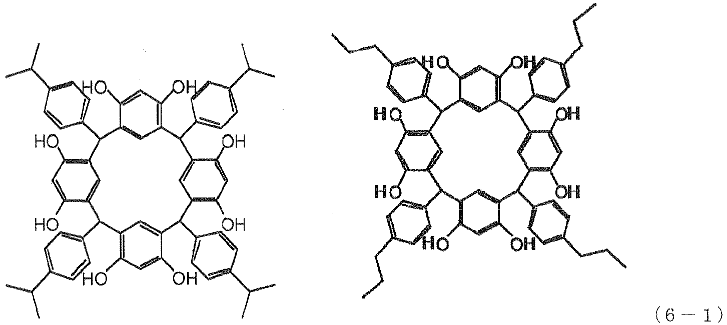

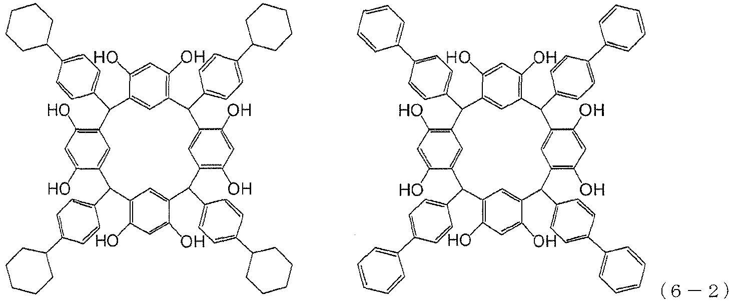

In the present invention, as the cyclic compound (B), the following are preferable. Cyclic compounds (B) represented by the following formulae (7) and (8) are preferable.

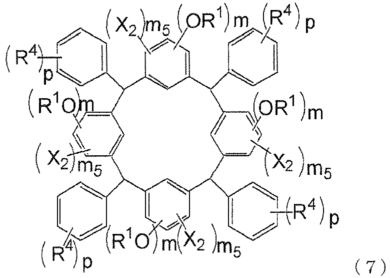

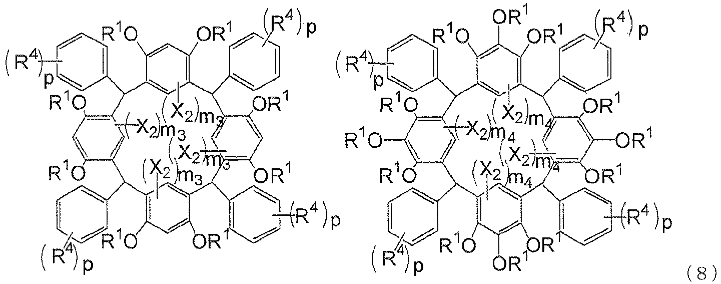

(In the formulae (7) and (8), R¹, R⁴, p and m are the same as above. However, at least one R¹ thereof is an acid dissociable functional group. X₂ is a hydrogen atom or a halogen atom, m₅ is an integer of 0 to 3, m+m₅=4, m₃ is 2, and m₄ is 1.)

In the present invention, as the cyclic compound (B), compounds represented by the following formula (9-1) or (9-2) are more preferable.

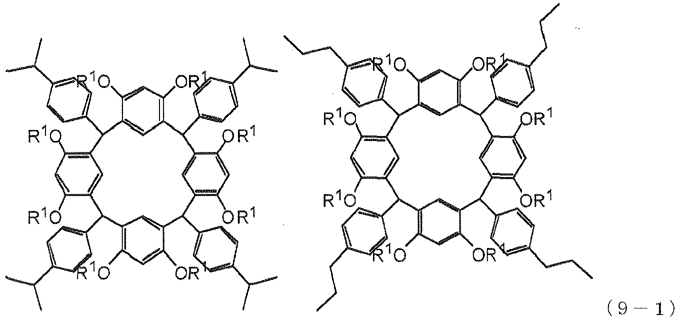

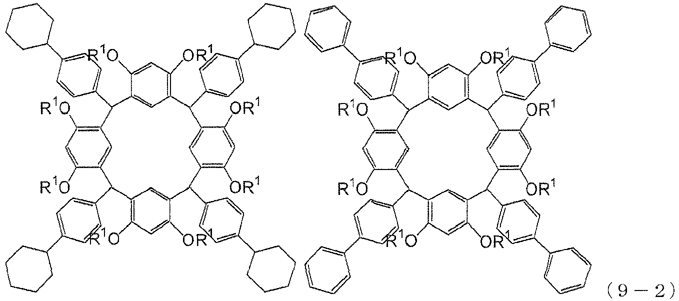

(In the formulae (9-1) and (9-2), R¹ is the same as above. However, at least one R¹ thereof is an acid dissociable functional group.)

The above acid dissociable functional group can be accordingly selected and used from ones suggested in a hydroxystyrene based resin, a (meth)acrylic acid based resin and the like used in a chemical amplification type resist composition for KrF or ArF. For example, a substituted methyl group, a 1-substituted ethyl group, a 1-substituted-n-propyl group, a 1-branched alkyl group, a silyl group, an acyl group, a 1-substituted alkoxymethyl group, a cyclic ether group, and an alkoxycarbonyl group are preferably included. The above acid dissociable functional group is preferable not to have any crosslinkable functional group.

As the substituted methyl group, a substituted methyl group of 2 to 20 carbons is normal, a substituted methyl group of 4 to 18 carbons is preferable, and a substituted methyl group of 6 to 16 carbons is further preferable. For example, a methoxymethyl group, a methylthiomethyl group, an ethoxymethyl group, an n-propoxymethyl group, an isopropoxymethyl group, an n-butoxymethyl group, a t-butoxymethyl group, a 2-methylpropoxy group, an ethylthiomethyl group, a methoxyethoxymethyl group, a phenyloxymethyl group, a 1-cyclopentyloxymethyl group, a 1-cyclohexyloxymethyl group, a benzylthiomethyl group, a phenacyl group, a 4-bromophenacyl group, a 4-methoxyphenacyl group, a piperonyl group, and substituent groups represented by the following formula (11) can be included.

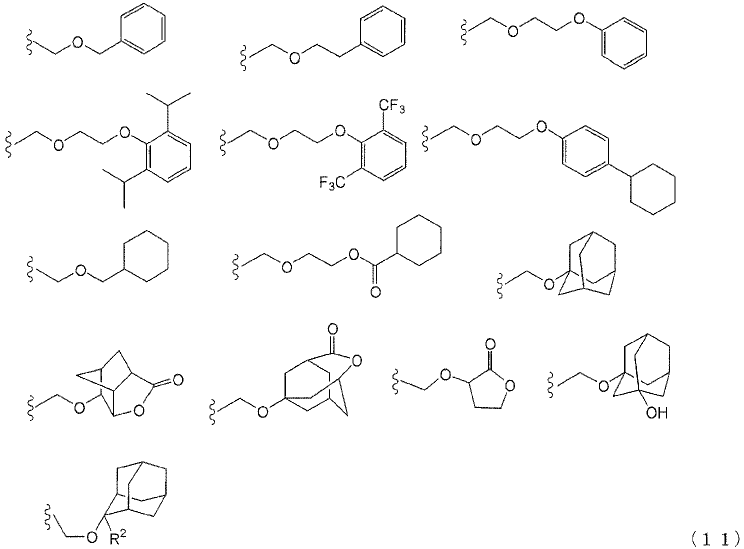

(In the formula (11), R² is a hydrogen atom or an alkyl group of 1 to 4 carbons. The alkyl groups of 1 to 4 carbons include a methyl group, an ethyl group, an isopropyl group, an n-propyl group, a t-butyl group, an n-butyl group and the like.)

As the 1-substituted ethyl group, a 1-substituted ethyl group of 3 to 20 carbons is normal, a 1-substituted ethyl group of 5 to 18 carbons is preferable, and a substituted ethyl group of 7 to 16 carbons is more preferable. For example, a 1-methoxyethyl group, a 1-methylthioethyl group, a 1,1-dimethoxyethyl group, a 1-ethoxyethyl group, a 1-ethylthioethyl group, a 1,1-diethoxyethyl group, an n-propoxyethyl group, an isopropoxyethyl group, an n-butoxyethyl group, a t-butoxyethyl group, a 2-methylpropoxyethyl group, a 1-phenoxyethyl group, a 1-phenylthioethyl group, a 1,1-diphenoxyethyl group, a 1-cyclopentyloxyethyl group, a 1-cyclohexyloxyethyl group, a 1-phenylethyl group, a 1,1-diphenylethyl group, and substituent groups represented by the following formula (12) can be included.

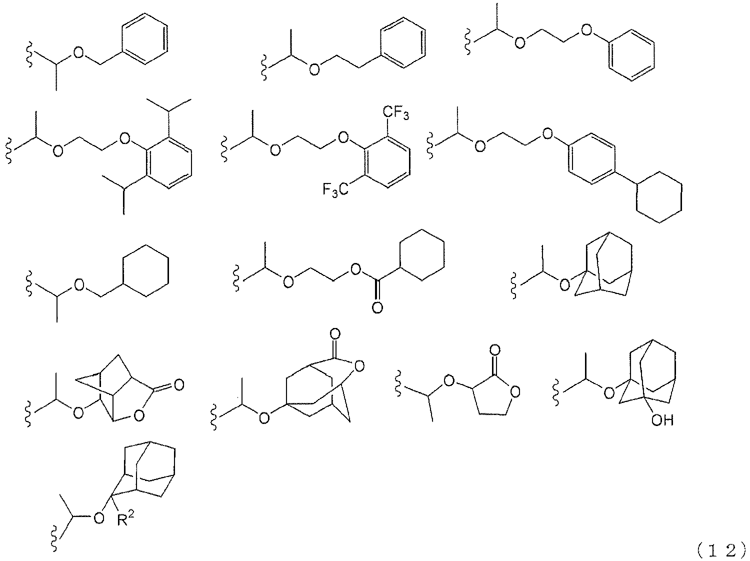

(In the formula (12), R² is the same as above.)

As the 1-substituted-n-propyl group, a 1-substituted-n-propyl group of 4 to 20 carbons is normal, a 1-substituted-n-propyl group of 6 to 18 carbons is preferable, and a 1-substituted-n-propyl group of 8 to 16 carbons is further preferable. For example, a 1-methoxy-n-propyl group and a 1-ethoxy-n-propylgroup can be included.

As the 1-branched alkyl group, a 1-branched alkyl group of 3 to 20 carbons is normal, a 1-branched alkyl group of 5 to 18 carbons is preferable, and a branched alkyl group of 7 to 16 carbons is further preferable. For example, an isopropyl group, a sec-butyl group, a tert-butyl group, a 1,1-dimethylpropyl group, a 1-methylbutyl group, a 1,1-dimethylbutyl group, a 2-methyladamantyl group, and a 2-ethyladamantyl group can be included.

As the silyl group, a silyl group of 1 to 20 carbons is normal, a silyl group of 3 to 18 carbons is preferable, and a silyl group of 5 to 16 carbons is further preferable. For example, a trimethylsilyl group, an ethyldimethylsilyl group, a methyldiethyl group, a triethylsilyl group, a tert-butyldimethylsilyl group, a tert-butyldiethylsilyl group, a tert-butyldiphenylsilyl group, a tri-tert-butylsilyl group and a triphenylsilyl group can be included.

As the acyl group, an acyl group of 2 to 20 carbons is normal, an acyl group of 4 to 18 carbons is preferable, and an acyl group of 6 to 16 carbons is further preferable. For example, an acetyl group, a phenoxyacetyl group, a propionyl group, a butyryl group, a heptanoyl group, a hexanoyl group, a valeryl group, a pivaloyl group, an isovaleryl group, a lauroyl group, an adamantylcarbonyl group, a benzoyl group and a naphthoyl group can be included.

As the 1-substituted alkoxymethyl group, a 1-substituted alkoxymethyl group of 2 to 20 carbons is normal, a 1-substituted alkoxymethyl group of 4 to 18 carbons is preferable, and a 1-substituted alkoxymethyl group of 6 to 16 carbons is further preferable. For example, a 1-cyclopentylmethoxymethyl group, a 1-cyclopentylethoxymethyl group, a 1-cyclohexylmethoxymethyl group, a 1-cyclohexylethoxymethyl group, a 1-cyclooctylmethoxymethyl group and a 1-adamantylmethoxymethyl group can be included.

As the cyclic ether group, a cyclic ether group of 2 to 20 carbons is normal, a cyclic ether group of 4 to 18 carbons is preferable, and a cyclic ether group of 6 to 16 carbons is further preferable. For example, a tetrahydropyranyl group, a tetrahydrofuranyl group, a tetrahydrothiopyranyl group, a tetrahydrothiofuranyl group, a 4-methoxytetrahydropyranyl group and a 4-methoxytetrahydrothiopyranyl group can be included.

As the alkoxycarbonyl group, an alkoxycarbonyl group of 2 to 20 carbons is normal, an alkoxycarbonyl group of 4 to 18 carbons is preferable, and an alkoxycarbonyl group of 6 to 16 carbons is more preferable. For example, a methoxycarbonyl group, an ethoxycarbonyl group, an n-propoxycarbonyl group, isopropoxycarbonyl group, an n-butoxycarbonyl group, a tert-butoxycarbonyl group or acid dissociable functional groups represented by the following formula (13) wherein n=0 can be included.

As the alkoxycarbonylalkyl group, an alkoxycarbonylalkyl group of 2 to 20 carbons is normal, an alkoxycarbonylalkyl group of 4 to 18 carbons is preferable, and an alkoxycarbonylalkyl group of 6 to 16 carbons is further preferable. For example, a methoxycarbonylmethyl group, an ethoxycarbonylmethyl group, an n-propoxycarbonylmethyl group, an isopropoxycarbonylmethyl group, an n-butoxycarbonylmethyl group or acid dissociable functional groups represented by the following formula (13) wherein n=1 to 4 can be included.

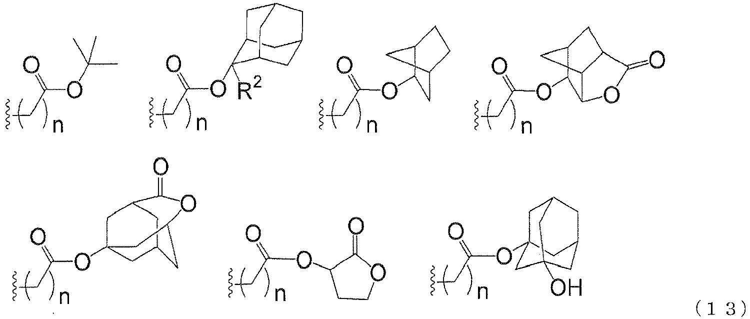

(In the formula (13), R² is the same as above, and n is an integer of 0 to 4.)

Among these acid dissociable functional groups, a substituted methyl group, a 1-substituted ethyl group, a 1-substituted alkoxymethyl group, a cyclic ether group, an alkoxycarbonyl group, and an alkoxycarbonylalkyl group are preferable, a substituted methyl group, a 1-substituted ethyl group, an alkoxycarbonyl group and an alkoxycarbonylalkyl group are more preferable due to high sensitivity, and a cycloalkane of 3 to 12 carbons, a lactone and an acid dissociable functional group with a structure selected from 6 to 12 aromatic rings are further more preferable.

The cycloalkane of 3 to 12 carbons may be monocyclic or polycyclic, and is more preferable to be polycyclic. Specifically, monocycloalkane, bicycloalkane, tricycloalkane, tetracycloalkane and the like are included, and more specifically, monocycloalkanes such as cyclopropane, cyclobutane, cyclopentane and cyclohexane, and polycycloalkanes such as an adamantine, norbornane, isobornane, tricyclodecane and tetracyclodecane are included. Among these, adamantine, tricyclodecane and tetracyclodecane are preferable, and adamantine and tricyclodecane are particularly preferable. The cycloalkanes of 3 to 12 carbons may have a substituent group. The lactones include a butyrolactone or a cycloalkane group of 3 to 12 carbons having a lactone group. The 6 to 12 aromatic rings include a benzene ring, a naphthalene ring, an anthracene ring, a phenanthrene ring, a pyrene ring and the like, a benzene ring and a naphthalene ring are preferable, and a naphthalene ring is particularly preferable.

Particularly, an acid dissociable functional group selected from the group consisting of each group represented by the following formula (10) is preferable in terms of high resolution.

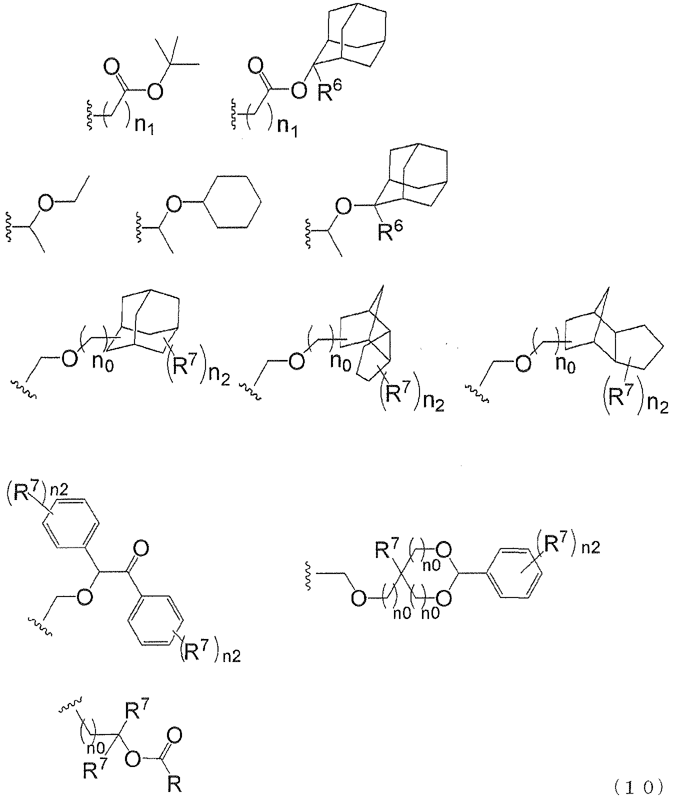

(In the formula (10), R⁶ is a hydrogen atom or a linear or branched alkyl group of 1 to 4 carbons, R⁷ is a hydrogen atom, a linear or branched alkyl group of 1 to 4 carbons, a cyano group, a nitro group, a heterocyclic group, a halogen atom or a carboxyl group, n₁ is an integer of 0 to 4, n₂ is an integer of 1 to 5, and n₀ is an integer of 0 to 4.)

Also, the acid dissociable functional group R¹ may be a substituent group consisting of a repeating unit represented by the following formula (14) and a terminal group represented by the following formula (15) or R¹ (R¹ is the same as above), as long as the effect of the present invention is not impaired.

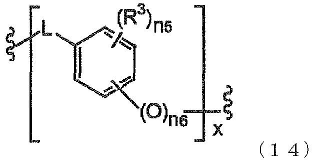

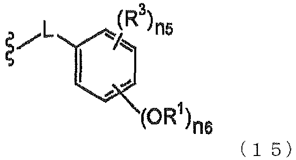

In the formula (14) and/or (15), R¹ is the same as above. L is the same as above, preferably a single bond, a methylene group, an ethylene group or a carbonyl group. A plurality of Q may be the same or different. n₅ is an integer of 0 to 4, n₆ is an integer of 1 to 3, x is an integer of 0 to 3, and 1≤n₅+n₆≤5 is met. A plurality of n₅, n₆ and x may be the same or different.

R³ is a substituent group selected from the group consisting of a halogen atom, an alkyl group, a cycloalkyl group, an aryl group, an aralkyl group, an alkoxy group, an aryloxy group, an alkenyl group, an acyl group, an alkoxycarbonyl group, an alkyloyloxy group, an aryloyloxy group, a cyano group, and a nitro group.

The halogen atoms include a chlorine atom, a bromine atom and an iodine atom; the alkyl groups include alkyl groups of 1 to 4 carbon atoms such as a methyl group, an ethyl group, a propyl group, an n-propyl group, an n-butyl group, an isobutyl group, a sec-butyl group and a tert-butyl group; the cycloalkyl groups include a cyclohexyl group, a norbornyl group, an adamantyl group and the like; the aryl groups include a phenyl group, a tolyl group, a xylyl group, a naphthyl group and the like; the aralkyl groups include a benzyl group, a hydroxybenzyl group, a dihydroxybenzyl group and the like; the alkoxy groups include alkoxy groups of 1 to 4 carbon atoms such as a methoxy group, an ethoxy group, a hydroxyethoxy group, a propoxy group, a hydroxypropoxy group, an isopropoxy group, an n-butoxy group, an isobutoxy group, a sec-butoxy group and a tert-butoxy group; the aryloxy groups include a phenoxy group and the like; the alkenyl groups include alkenyl groups of 2 to 4 carbon atoms such as a vinyl group, a propenyl group, an allyl group and a butenyl group; the acyl groups include aliphatic acyl groups of 1 to 6 carbon atoms such as a formyl group, an acetyl group, a propionyl group, a butyryl group, a valeryl group, an isovaleryl group and a pivaloyl group, and aromatic acyl groups such as a benzoyl group and a toluoyl group; the alkoxycarbonyl groups include alkoxycarbonyl groups of 2 to 5 carbon atoms such as a methoxycarbonyl group, an ethoxycarbonyl group, a propoxycarbonyl group, an isopropoxycarbonyl group, an n-butoxycarbonyl group, an isobutoxycarbonyl group, a sec-butoxycarbonyl group and a tert-butoxycarbonyl group; the alkyloyloxy groups include an acetoxy group, a propionyloxy group, a butyryloxy group, an isobutyryloxy group, a valeryloxy group, an isovaleryloxy group and a pivaloyloxy group; the aryloyloxy groups include a benzoyloxy group and the like. A plurality of R³ may be the same or different.

In the present invention, the acid dissociable functional group refers to a characteristic group for cleaving in the presence of acid to generate an alkali soluble group. The alkali soluble groups include a phenolic hydroxyl group, a carboxyl group, a sulfonate group and a hexafluoroisopropanol group, a phenolic hydroxyl group and a carboxyl group are preferable, and a phenolic hydroxyl group is particularly preferable. The acid dissociable functional group is preferable to have the property of initiating a chained cleavage reaction in the presence of acid, in order to allow for pattern formation with further high sensitivity and high resolution.

These cyclic compounds may be independent, or may be mixed with two or more kinds. Also, the cyclic compounds may be ones containing various surfactants, various crosslinkers, various acid generators, various stabilizers and the like.

The organic solvent used in the present invention is not particularly limited as long as it is an organic solvent which is not arbitrarily blended with water, but an organic solvent which can be safely applied to a semiconductor production process is preferable. The amount of the organic solvent used is normally about 1 to 100 times by weight based on polymer used.

Specific examples of the solvent used include ethers such as diethyl ether and diisopropyl ether, esters such as ethyl acetate, n-butyl acetate and isoamyl acetate, ketones such as methyl ethyl ketone, methyl isobutyl ketone, ethyl isobutyl ketone, cyclohexanone, cyclopentanone, 2-heptanone and 2-pentanone, glycol ether acetates such as ethylene glycol monoethyl ether acetate, ethylene glycol monobutyl ether acetate, propylene glycol monomethyl ether acetate and propylene glycol monoethyl ether acetate, aliphatic hydrocarbons such as n-hexane and n-heptane, aromatic hydrocarbons such as toluene and xylene, halogenated hydrocarbons such as methylene chloride and chloroform. Among these, toluene, 2-heptanone, cyclohexanone, cyclopentanone, methyl isobutyl ketone, ethyl acetate and the like are preferable, and cyclohexanone is particularly preferable. These solvents may be used each independently, or may be used in a mixture of two or more kinds.

The liquid to be used for contacting with the solution containing the cyclic compound and the organic solvent is water or an acidic aqueous solution. The acidic aqueous solution used in the present invention is accordingly selected from aqueous solutions having commonly known organic and inorganic based compounds dissolved in water. For example, aqueous solutions having mineral acids such as hydrochloric acid, sulfuric acid, nitric acid and phosphoric acid dissolved in water, or aqueous solutions having organic acids such as acetic acid, propionic acid, oxalic acid, malonic acid, succinic acid, fumaric acid, maleic acid, tartaric acid, citric acid, methanesulfonic acid, phenolsulfonic acid, p-toluenesulfonic acid, and trifluoroacetic acid are included. These acidic aqueous solutions may be used each independently, or may be used in combination of two kinds or more. Among these acidic aqueous solutions, aqueous solutions of carboxylic acids such as sulfuric acid, oxalic acid, tartaric acid and citric acid are preferable, aqueous solutions of oxalic acid, tartaric acid and citric acid are further preferable, and an aqueous solution of oxalic acid is particularly preferable. It is thought that polycarboxylic acids such as oxalic acid, tartaric acid and citric acid can remove more metals due to the metal ion coordination and the chelating effect generated. Also, water to be used here is, according to the object of the present invention, preferably one with a low metal content, for example ion exchange water and the like.

Although the pH of an acidic aqueous solution used in the present invention is not particularly limited, when the acidity of an aqueous solution becomes too high, a cyclic compound may be adversely impacted, which is not preferable. The pH range is normally about 0 to 5, more preferably about pH 0 to 3.

Although the amount of an acidic aqueous solution used in the present invention is not particularly limited, when the amount is too small, the number of extractions for metal removal is required to be more, and conversely when the amount of an aqueous solution is too large, the entire liquid amount becomes large, which may generate a problem upon operation. The amount of an aqueous solution used is normally 10 to 200 parts by mass, preferably 20 to 100 parts by mass based on 100 parts by mass of a solution of a cyclic compound dissolved in an organic solvent.

In the present invention, an extraction treatment is conducted by contacting a solution containing the above cyclic compound and the above organic solvent with water or the above acidic aqueous solution. The temperature upon conducting the extraction treatment ranges normally from 20 to 90 °C, and preferably from 30 to 80 °C. In the extraction operation, the metal content contained in a solution containing a cyclic compound and an organic solvent is transferred to the water phase by mixing it better through stirring and the like, for example.

Afterwards, by leaving it to stand, the solution phase containing a cyclic compound and an organic solvent and the water phase are separated. Then, by decantation and the like for example, a solution containing a cyclic compound and an organic solvent is recovered. Although the time of leaving it to stand is not particularly limited, when the time of leaving it to stand is too short, separation between the solution phase containing a cyclic compound and an organic solvent and the water phase become poor, which is not preferable. The time of leaving it to stand is normally not less than 10 minutes, and preferably not less than 30 minutes.
The extraction treatment may be once, and it is also effective to repeat operations such as mixing, leaving to stand and separating more than once.

When such an extraction treatment is conducted by using an acidic aqueous solution, after conducting the extraction treatment, a solution containing a cyclic compound and an organic solvent which has been extracted and recovered from the aqueous solution is preferably further subjected to an extraction treatment using water. The water to be used here is, according to the object of the present invention, preferably one with a low metal content, for example ion exchange water and the like. This extraction treatment may be once, but it is also effective to repeat operations such as mixing, leaving to stand and separating more than once. Moreover, conditions such as the usage ratio of the both in the extraction treatment, temperature and time are not particularly limited, but may be the same with the case of the above contact process with an acidic aqueous solution.

The water content mixed in the solution thus obtained containing a cyclic compound and an organic solvent can be easily removed by conducting operations such as distillation under reduced pressure. Also, by adding an organic solvent if required, the concentration of a cyclic compound can be adjusted to be an arbitrary concentration.

A method for obtaining a cyclic compound only from the resultant solution containing an cyclic compound and an organic solvent can be conducted by publicly known methods such as removal under reduced pressure, separation by reprecipitation, and combinations thereof, for example.

### EXAMPLES

Below, embodiments of the present invention will be specifically described with reference to examples. However, the present invention is not limited to these examples. In the following synthesis examples, the structure of each compound is confirmed with ¹H-NMR measurement.

### <Synthesis Example> Synthesis of Cyclic Compound (CR-1)

To a four-neck flask (1000 mL) equipped with a dropping funnel, a Dimroth condenser tube, a thermometer and a stirring blade, sufficiently dried and substituted with nitrogen, resorcinol (22 g, 0.2 mol) made by Kanto Chemical Co., 4-cyclohexylbenzaldehyde (46.0 g, 0.2 mol) and dehydrated ethanol (200 mL) were charged under a nitrogen gas stream, so as to prepare an ethanol solution. This solution was heated by a mantle heater to 85°C while stirring. Next, 75 mL of concentrated hydrochloric acid (35 %) was dropped through the dropping funnel for 30 minutes, and then continuously stirred at 85°C for 3 hours. After the reaction terminated, it was left to cool to room temperature, and then cooled in an ice bath. After leaving it to stand for 1 hour, the objective coarse light yellow crystal was produced, and this was filtered. The coarse crystal was washed with 500 ml of methanol twice, filtered and vacuum dried to yield the objective product (hereinafter, referred to as CR-1) (50 g, 91% yield).
The structure of this compound showed, as a result of analyzing with LC-MS, the molecular weight of the objective product to be 1121. Moreover, the chemical shift values (δ ppm, TMS standard) of ¹H-NMR in a heavy dimethyl sulfoxide solvent were 1.2 to 1.4 (m, 20H), 1.7 to 1.9 (m, 20H), 2.2 to 2.4 (m, 4H), 5.5, 5.6 (d, 4H), 6.0 to 6.8 (m, 24H), 8.4 to 8.5 (m, 8H).

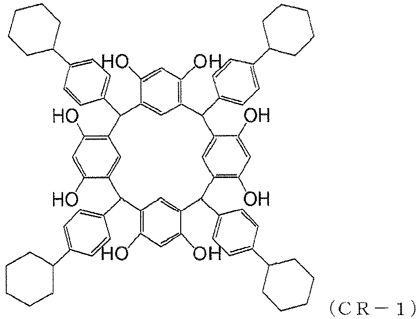

### <Examples> Production of Cyclic Compound with Reduced Metal Content (Example 1)

To a 1000 mL four-neck flask (without base plate), 650 g of a solution (1.7 wt%) having CR-1 dissolved in cyclohexanone was charged, and heated to 75°C while stirring. Next, 130 g of an oxalic acidic aqueous solution (pH 0.9) was added, stirred for 3 minutes, and then left to stand for 1 hour. As the oil phase and the water phase were separated thereby, the water phase was removed. To the resultant oil phase, 130 g of ultrapure water was charged, stirred for 3 minutes, and then left to stand for 1 hour to remove the water phase. By repeating this operation four times, a solution of a cyclic compound in cyclohexanone was obtained.

### (Example 2)

A solution of a cyclic compound in cyclohexanone was obtained by the same process, except that 130 g of a citric acidic aqueous solution (pH 1.8) was charged instead of charging 130 g of the oxalic acidic aqueous solution (pH 0.9) in Example 1.

### (Example 3)

A solution of a cyclic compound in cyclohexanone was obtained by the same process, except that 130 g of a tartaric acidic aqueous solution (pH 1.8) was charged instead of charging 130 g of the oxalic acidic aqueous solution (pH 0.9) in Example 1.

### (Example 4)

A solution of a cyclic compound in cyclohexanone was obtained by the same process, except that 130 g of an acetic acidic aqueous solution (pH 2.8) was charged instead of charging 130 g of the oxalic acidic aqueous solution (pH 0.9) in Example 1.

### (Example 5)

A solution of a cyclic compound in cyclohexanone was obtained by the same process, except that 130 g of ion exchange water was charged instead of charging 130 g of the oxalic acidic aqueous solution (pH 0.9) in Example 1.

### <Comparative Example> Production of Cyclic Compound with Reduced Metal Content by Ion Exchange Resin

### (Comparative Example 1)

25 g of an ion exchange resin (Mitsubishi Chemical DIAION: SMT100-MIEX Resin) was swollen with cyclohexanone, then filled in a Teflon® column, and solvent substituted by feeding 500 mL of 1,3-dioxolan. Next, by feeding 500 g of a solution (1.7 wt%) having CR-1 dissolved in 1,3-dioxolan, a cyclic compound was obtained.

For the solution of CR-1 in cyclohexanone before the process, and the cyclic compounds obtained in Examples 1 to 5 and Comparative Example 1, the contents of various metals were measured by ICP-MS. The measurement results are shown in Table 1.

**[Table 1]**

| | Metal Content (ppb) | | | | | | |
|---|---|---|---|---|---|---|---|
| | Na | Mg | K | Cr | Fe | Cu | Zn |
| Before Process | 55.6 | 1.1 | 2.3 | 28.8 | 132.0 | 10.0 | 11.5 |
| Example 1 | ≤0.2 | ≤0.2 | ≤0.2 | ≤0.2 | ≤0.2 | ≤0.2 | ≤0.2 |
| Example 2 | ≤0.2 | ≤0.2 | ≤0.2 | 3.4 | 1.4 | ≤0.2 | ≤0.2 |
| Example 3 | ≤0.2 | ≤0.2 | ≤0.2 | 6.7 | 1.4 | ≤0.2 | ≤0.2 |
| Example 4 | ≤0.2 | ≤0.2 | ≤0.2 | 8.5 | 52.5 | ≤0.2 | ≤0.2 |
| Example 5 | ≤0.2 | ≤0.2 | 0.3 | 8.5 | 92.6 | 0.3 | ≤0.2 |
| Comparative Example 1 | ≤0.2 | 0.5 | 1.0 | 16.7 | 132.0 | 1.2 | 0.4 |

### INDUSTRIAL APPLICABILITY

The present invention can industrially advantageously purify a cyclic compound with a reduced metal content.

## Claims

1. A purification method for a cyclic compound, including a step of contacting a solution containing a cyclic compound represented by the following formula (1) and an organic solvent with water or an acidic aqueous solution. 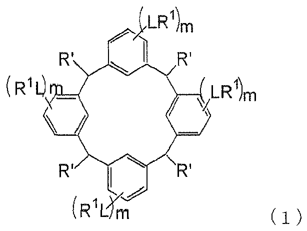 (In the formula (1), L is independently a divalent group selected from the group consisting of a single bond, a linear or branched alkylene group of 1 to 20 carbons, a cycloalkylene group of 3 to 20 carbons, an arylene group of 6 to 24 carbons, -O-, - OC(=O)-, -OC(=O)O-, -N(R⁵)-C(=O)-, -N(R⁵)-C(=O)O-, -S-, -SO-, -SO₂- and combinations thereof, R¹ is independently an acid dissociable functional group selected from the group consisting of an alkyl group of 1 to 20 carbons, a cycloalkyl group of 3 to 20 carbons, an aryl group of 6 to 20 carbons, an alkoxyl group of 1 to 20 carbons, a cyano group, a nitro group, a hydroxyl group, a heterocyclic group, a halogen atom, a carboxyl group, an acyl group of 2 to 20 carbons, an alkylsilyl group of 1 to 20 carbons, a substituted methyl group of 2 to 20 carbons, a 1-substituted ethyl group of 3 to 20 carbons, a 1-substituted-n-propyl group of 4 to 20 carbons, a 1-branched alkyl group of 3 to 20 carbons, a silyl group 1 to 20 carbons, an acyl group of 2 to 20 carbons, a 1-substituted-alkoxyalkyl group of 2 to 20 carbons, a cyclic ether group of 2 to 20 carbons, an alkoxycarbonyl group of 2 to 20 carbons and an alkoxycarbonylalkyl group, or a hydrogen atom, R' is independently an alkyl group of 2 to 20 carbons or a group represented by the following formula 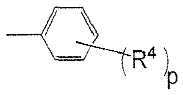 wherein, R⁴ is independently a functional group selected from the group consisting of an alkyl group of 1 to 20 carbons, a cycloalkyl group of 3 to 20 carbons, an aryl group of 6 to 20 carbons, an alkoxy group of 1 to 20 carbons, a cyano group, a nitro group, a hydroxyl group, a heterocyclic group, a halogen atom, a carboxyl group and an alkylsilyl group of 1 to 20 carbons, or an acid dissociable functional group selected from the group consisting of a substituted methyl group of 2 to 20 carbons, a 1-substituted ethyl group of 3 to 20 carbons, a 1-substituted-n-propyl group of 4 to 20 carbons, a 1-branched alkyl group of 3 to 20 carbons, a silyl group 1 to 20 carbons, an acyl group of 2 to 20 carbons, a 1-substituted-alkoxyalkyl group of 2 to 20 carbons, a cyclic ether group of 2 to 20 carbons, an alkoxycarbonyl group of 2 to 20 carbons and an alkoxycarbonylalkyl group, R⁵ is a hydrogen atom or an alkyl group of 1 to 10 carbons, m is an integer of 1 to 4, and p is an integer of 0 to 5.)

2. A purification method according to Claim 1, further including a step of conducting an extraction treatment of the cyclic compound.

3. A purification method according to Claim 2, further including a step of separating the solution phase containing the cyclic compound and the organic solvent and the water phase after the extraction treatment.

4. A purification method according to Claim 3, further including a step of recovering a solution containing the cyclic compound and the organic solvent.

5. A purification method according to any of Claims 1 to 4, wherein the acidic aqueous solution is an aqueous solution of one or more kinds of mineral acids selected from the group consisting of hydrochloric acid, sulfuric acid, nitric acid and phosphoric acid.

6. A purification method according to any of Claims 1 to 4, wherein the acidic aqueous solution is an aqueous solution of one or more kinds of organic acids selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid and trifluoroacetic acid.

7. A purification method according to any of Claim 6, wherein the acidic aqueous solution is an aqueous solution of at least one kind of polycarboxylic acid selected from oxalic acid, tartaric acid and citric acid.

8. A purification method according to Claim 7, wherein the acidic aqueous solution is an aqueous solution of oxalic acid.

9. A purification method according to any of Claims 1 to 8, wherein the organic solvent is toluene, 2-heptanone, cyclohexanone, cyclopentanone, methyl isobutyl ketone, or ethyl acetate.

10. A purification method according to Claim 9, wherein the organic solvent is cyclohexanone.

11. A purification method according to any of Claims 1 to 10, wherein the cyclic compound represented by the formula (1) is selected from the group consisting of each compound represented by the following formula (2). 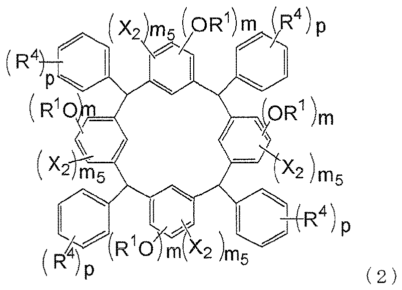 (In the formula (2), R¹, R⁴, p and m are the same as above. X₂ is a hydrogen atom or a halogen atom, m₅ is an integer of 0 to 3, and m+m₅=4.)

12. A purification method according to Claim 11, wherein the compound represented by the formula (2) is selected from the group consisting of each compound represented by the following formula (3). 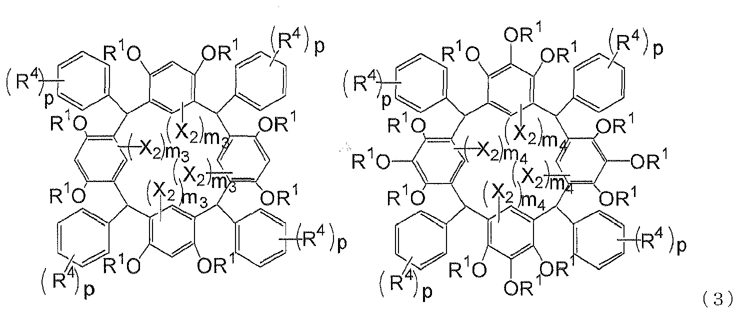 (In the formula (3), R¹, R⁴, X₂ and p are the same as above. m₃ is 2, and m₄ is 1.)

13. A purification method according to Claim 11, wherein the compound represented by the formula (2) is selected from the group consisting of each compound represented by the following formula (4). 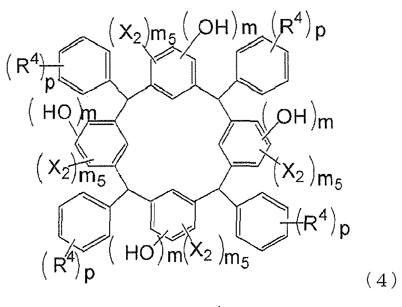 (In the formula (4), R⁴, X₂, m, m₅ and p are the same as above.)

14. A purification method according to Claim 13, wherein the compound represented by the formula (4) is selected from the group consisting of each compound represented by the following formula (5). 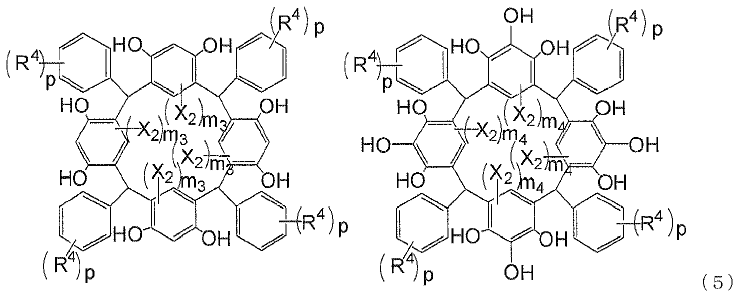 (In the formula (5), X₂, R⁴, m₃, m₄ and p are the same as above.)

15. A purification method according to Claim 14, wherein the compound represented by the formula (5) is selected from the group consisting of each compound represented by the following formulae (6-1) and (6-2).

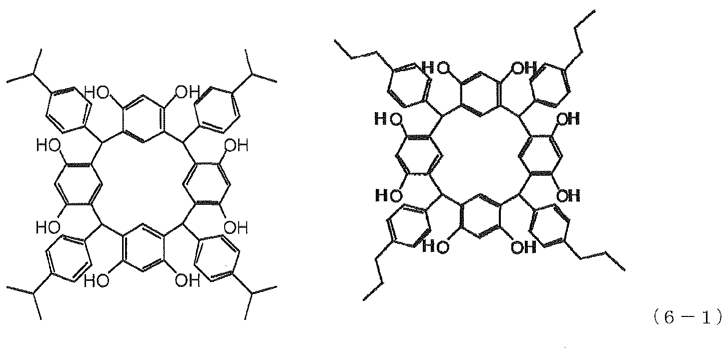

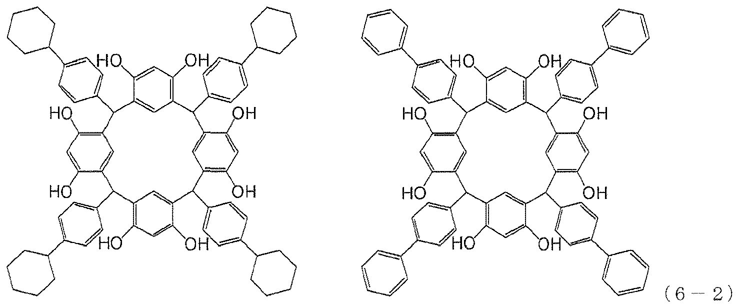

16. A purification method according to any of Claims 1 to 10, wherein the cyclic compound represented by the formula (1) is selected from the group consisting of each compound represented by the following formula (7). 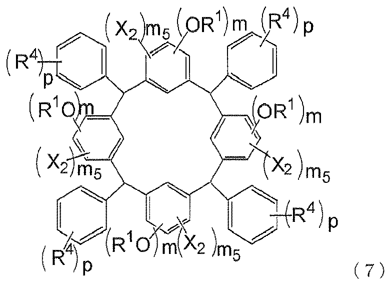 (In the formula (7), R¹, R⁴, p and m are the same as above. However, at least one R¹ thereof is an acid dissociable functional group. X₂ is a hydrogen atom or a halogen atom, m₅ is an integer of 0 to 3, and m+m₅=4.)

17. A purification method according to Claim 16, wherein the compound represented by the formula (7) is selected from the group consisting of each compound represented by the following formula (8). 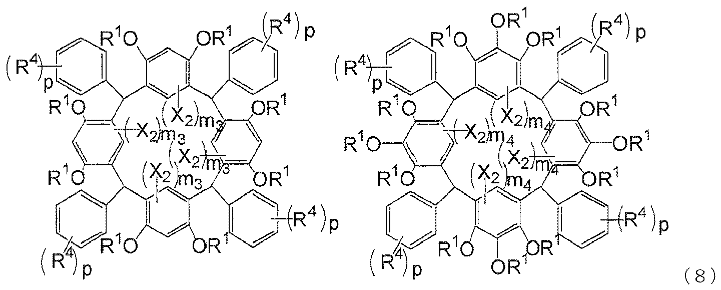 (In the formula (8), R¹, R⁴, X₂ and p are the same as above, with the proviso that at least one R¹ thereof is an acid dissociable functional group. m₃ is 2, and m₄ is 1.)

18. A purification method according to Claim 17, wherein the compound represented by the formula (8) is selected from the group consisting of each compound represented by the following formulae (9-1) and (9-2). 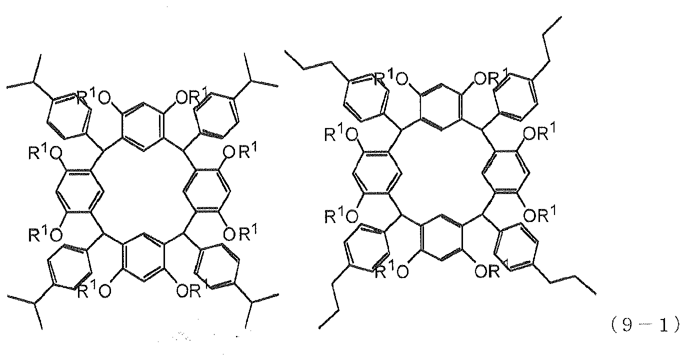 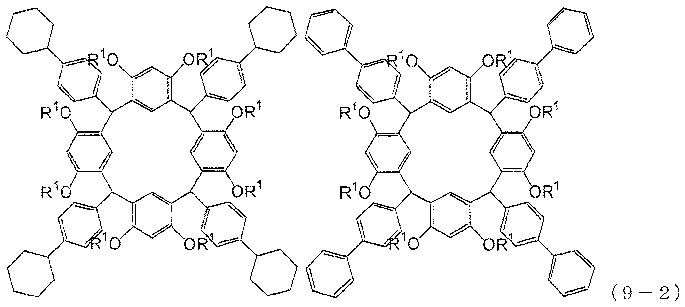 (In the formulae (9-1) and (9-2), R¹ is the same as above, with the proviso that at least one R¹ thereof is an acid dissociable functional group.)
